# EUROPÄISCHE PATENTSCHRIFT

(11) **EP 0 612 539 B1**
(45) Veröffentlichungstag und Bekanntmachung des Hinweises auf die Patenterteilung: **26.06.1996**
(21) Anmeldenummer: 94102852.4
(22) Anmeldetag: 25.02.1994
(51) Int. Cl.: A61N 5/06

(54) **Bräunungsgerät**
Sun bed
Appareil de bronzage

(30) Priorität: 27.02.1993 DE 4306100
(43) Veröffentlichungstag der Anmeldung: 31.08.1994
(73) Patentinhaber: König, Klaus, Dipl.-Ing. (FH), D-91452 Wilhermsdorf (DE)
(72) Erfinder: König, Klaus, Dipl.-Ing. (FH), D-91452 Wilhermsdorf (DE)
(74) Vertreter: Kessel, Egbert, Dipl.-Ing.

(56) Entgegenhaltungen:
- EP-A- 0 367 032
- DE-C- 116 294
- US-A- 4 907 132

## Beschreibung

Die Erfindung betrifft ein Bräunungsgerät, das aus einer Liege, einem Fluter und ggf. einem Gesichtsbräuner besteht, die einen Bräunungstunnel bilden, wobei aus dem Innenraum der Liege, des Fluters und ggf. des Gesichtsbräuners UV-Strahlung in den Bräunungstunnel überführt wird und eine zentrale Strahlenerzeugung vorgesehen ist, die entfernt von den den Bräunungstunnel begrenzenden Strahlenaustrittsflächen angeordnet ist, und wobei die zentrale Strahlenerzeugung mit dem Innenraum der Liege, des Fluters und ggf. des Gesichtsbräuners über Lichtwellenleiter verbunden ist.

Aus der Praxis ist ein Bräunungsgerät bekannt, bei dem die UV-Strahlung in der Liege und im Fluter durch eine Vielzahl von Röhren und im Gesichtsbräuner durch Hochdruckbrenner erzeugt wird. Diese Art der Strahlenerzeugung führt zu einer starken Erhitzung der Bräunungsgeräte, wobei die Abwärme am Körper in Erscheinung tritt. Es ist deshalb eine besondere Gerätekühlung erforderlich, die mittels eines Luftstroms erzeugt wird, was zu Staubaufwirbelungen und einer unerwünschten Erhöhung der Raumtemperatur führt. Als besonders ungünstig hat sich jedoch der bei den bekannten Bräunungsgeräten notwendige hohe Anschlußwert (etwa 16kW/Gerät) erwiesen; Sonnenstudios, in denen mehrere solcher Geräte installiert werden, müssen daher elektrisch nachgerüstet werden.

Der Nachteil einer zu starken Erhitzung ist durch ein aus der US 4,907,132 bekanntes Bräunungsgerät teilweise behoben worden, bei dem eine entfernt von den Strahlenaustrittsflächen angeordnete zentrale Strahlenerzeugung vorgesehen ist, die mit der Liege über Lichtwellenleiter verbunden ist. Durch die räumliche Trennung der Strahlenerzeugung und des Bräunungsgeräts wirkt sich die bei der Erzeugung der UV-Strahlen auftretende Wärme nicht auf das Gerät aus, so daß es keiner Gerätekühlung bedarf und ein größerer Bräunungskomfort erzielt wird. Eine wesentliche Verringerung des hohen Anschlußwerts konnte jedoch auch mit dieser Maßnahme nicht erzielt werden.

Der Erfindung liegt die Aufgabe zugrunde, ein Bräunungsgerät zu schaffen, das kaum Abwärme am Körper auftreten läßt und mit einem geringeren Anschlußwert als die bekannten Bräunungsgeräte auskommt.

Diese Aufgabe wird durch die Merkmale des Patentanspruchs 1 gelöst. Erfindungsgemäß ist vorgesehen, daß die Lichtwellenleiter aus den den Strahlenaustrittsflächen gegenüberliegenden Wandbereichen der Liege, des Fluters und ggf. des Gesichtsbräuners vorstehen, wobei jedem Lichtwellenleiterende eine Einrichtung zugeordnet ist, die eine großflächige Ausbreitung des austretenden Strahlenbündels bei gleichmäßiger Verteilung der Strahlen bewirkt.

Die Verwendung eines über Lichtwellenleiter zugeführten, großflächigen ausgebreiteten und gleichmäßig verteilten Strahlenbündels schafft die Möglichkeit, mit einem geringeren Aufwand bei der Strahlenerzeugung auszukommen, so daß in Verbindung mit dem Fortfall der Gerätekühlung auch mit einem erheblichen reduzierten Anschlußwert auszukommen ist.

Vorteilhafte Weiterbildungen der Erfindung ergeben sich aus den Merkmalen der Patentansprüche 2-11. - Gemäß einem ersten Ausführungsbeispiel der Erfindung ist die Einrichtung als Reflektoreinheit ausgebildet, die aus einem Hauptreflektor und einem Gegenreflektor besteht. Diese Ausgestaltung zeichnet sich vor allem durch bauliche Einfachheit und Wartungsfreundlichkeit aus.

In weiterer Ausgestaltung der Erfindung ist der Hauptreflektor gewölbt und mit seiner konkaven Fläche der Strahlenaustrittsfläche zugewandt, wobei der Durchgang des Lichtwellenleiters durch den Hauptreflektor in dessen optischer Achse angeordnet ist, während der Gegenreflektor in dem vom Hauptreflektor umschlossenen Raum angeordnet, gewölbt und mit seiner konkaven Fläche dem Hauptreflektor zugewandt ist.

Dabei hat es sich als besonders günstig erwiesen, wenn der Gegenreflektor auf der optischen Achse des Hauptreflektors gegenüber dem Durchgang des Lichtwellenleiters und im bzw. nahe dem Brennpunkt des Hauptreflektors angeordnet ist.

Eine Verbesserung der Ausbreitung des aus dem Lichtwellenleiter austretenden Strahlenbündels läßt sich dadurch erreichen, daß gemäß einem weiteren Merkmal der Erfindung zwischen Haupt- und Gegenreflektor am Ende des Lichtwellenleiters eine Linse vorgesehen ist. Diese Linse kann am Ende des Lichtwellenleiters angeschliffen sein, wobei es sich dann empfiehlt, die Fläche des Gegenreflektors größer als bei dem zuvor dargestellten Ausführungsbeispiel auszubilden.

Bei einem anderen Ausführungsbeispiel der Erfindung ist die Einrichtung als Linsensystem ausgebildet.

Schließlich läßt sich eine weitere Verbesserung des Bräunungskomforts dadurch erzielen, daß gemäß einem besonders vorteilhaften Merkmal der Erfindung der vom Hauptreflektor und der Strahlenaustrittsfläche umschlossene Raum der Liege mit einem Gel gefüllt und die Strahlenaustrittfläche als transparente verformbare Folie ausgebildet ist.

In der Zeichnung ist ein bevorzugtes Ausführungsbeispiel der Erfindung dargestellt.

Es zeigen
- Fig. 1: eine schematische Ansicht eines Bräunungsgeräts,

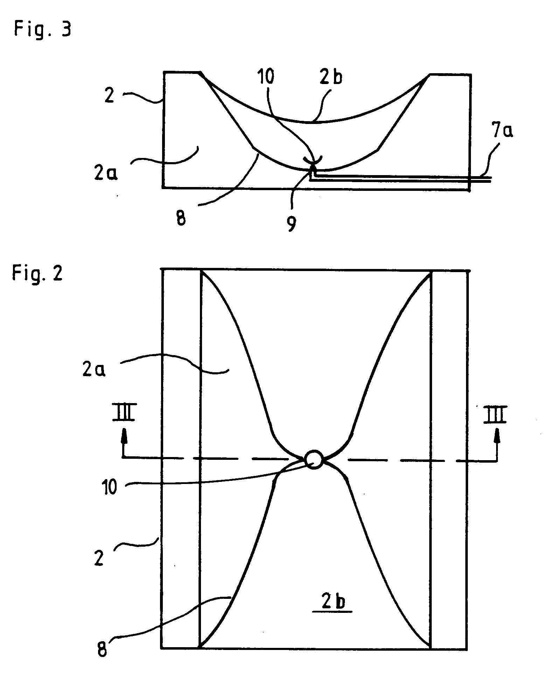

- Fig. 2: eine Draufsicht der Liege,
- Fig. 3: eine Schnittansicht der Liege nach Linie III - III in Fig. 2,

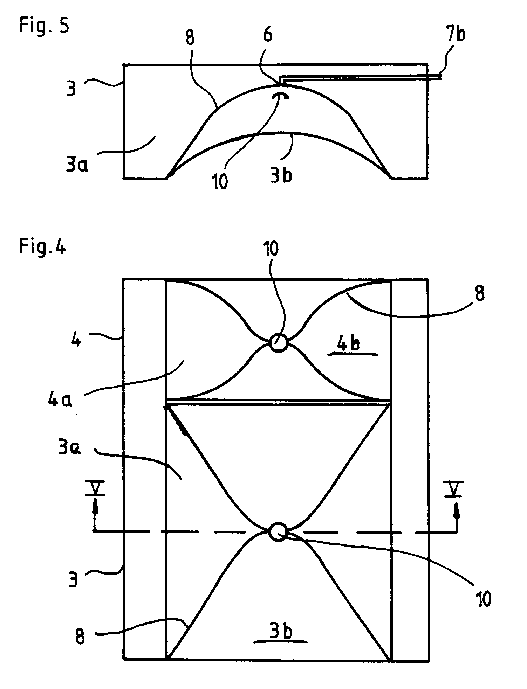

- Fig. 4: eine Unteransicht des Fluters mit Gesichtsbräuner,
- Fig. 5: eine Schnittansicht des Fluters nach Linie V - V in Fig. 4,

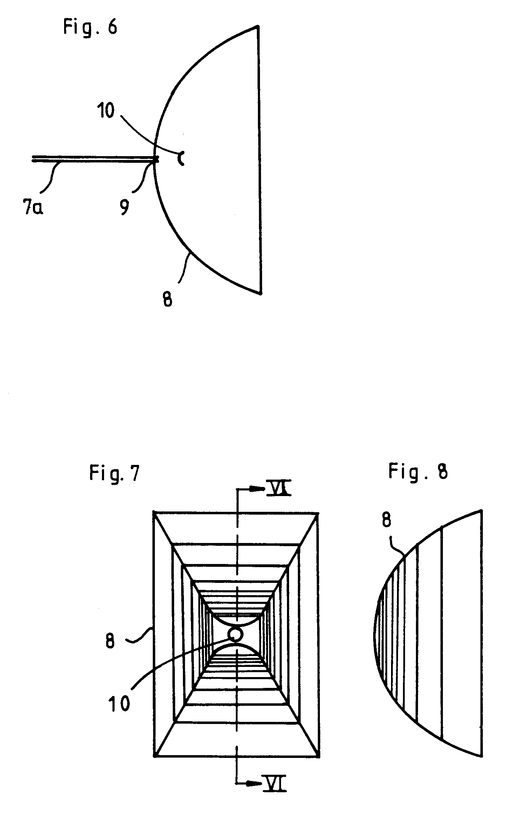

- Fig. 6: eine Schnittansicht der Reflektoreinheit nach Linie VI - VI in Fig. 7,
- Fig. 7: eine Innenansicht des Hauptreflektors und
- Fig. 8: eine Seitenansicht des Hauptreflektors.

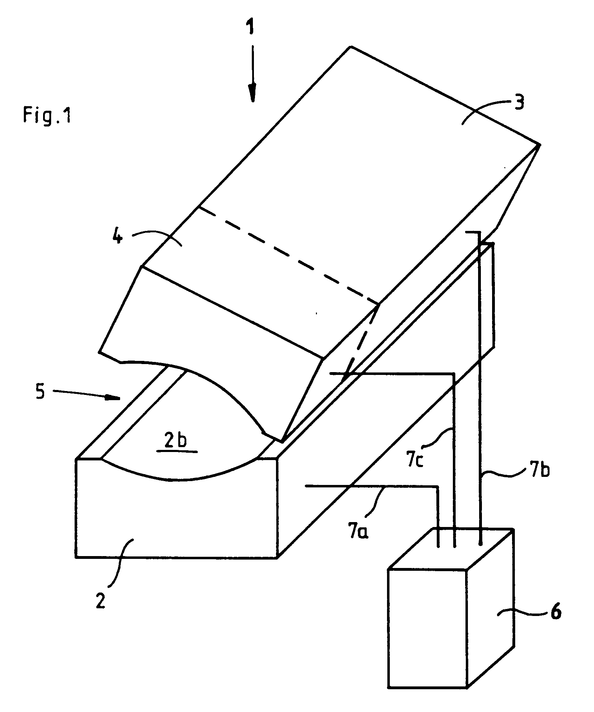

Fig. 1 zeigt ein Bräunungsgerät 1, das aus einer Liege 2, einem Fluter 3 und einem Gesichtsbräuner 4 besteht; die Liege 2 einerseits sowie der Fluter 3 und der Gesichtsbräuner 4 andererseits umschließen einen Bräunungstunnel 5. Von einer zentralen Strahlenerzeugung 6, die von dem Bräunungsgerät 1 räumlich getrennt ist, führen Lichtwellenleiter 7a, 7b und 7c in den Innenraum 2a, 3a und 4a der Liege 2, des Fluters 3 und des Gesichtsbräuners 4.

Jeder der Lichtwellenleiter 7a, 7b und 7c mündet in einem Hauptreflektor 8, der sich im Innenraum 2a, 3a und 4a der Liege 2, des Fluters 3 und des Gesichtsbräuners 4 befindet. Der Hauptreflektor 8 ist gewölbt, wobei seine konkave Fläche jeweils der Strahlenaustrittsfläche 2b, 3b und 4b der Liege 2, des Fluters 3 und des Gesichtsbräuners zugewandt ist; er erstreckt sich über die gesamte Fläche der Liege 2, des Fluters 3 und des Gesichtsbräuners 4.

Die Lichtwellenleiter 7a, 7b und 7c durchdringen den Hauptreflektor 8 von der konvexen Seite in seiner optischen Achse, wobei der Durchgang mit 9 bezeichnet ist. In dem vom Hauptreflektor 8 umschlossenen Raum ist auf dessen optischer Achse etwa in seinem Brennpunkt ein Gegenreflektor 10 angeordnet, der sich somit genau gegenüber dem Ende der Lichtwellenleiter 7a, 7b und 7c befindet und mit seiner konvexen Fläche dem Hauptreflektor 8 zugewandt ist.

### Bezugszeichenliste

- 1: Bräunungsgerät
- 2: Liege
- 2a: Innenraum
- 2b: Strahlenaustrittsfläche
- 3: Fluter
- 3a: Innenraum
- 3b: Strahlenaustrittsfläche
- 4: Gesichtsbräuner
- 4a: Innenraum
- 4b: Strahlenaustrittsfläche
- 5: Bräunungstunnel
- 6: zentrale Strahlenerzeugung
- 7a) 7b) 7c): Lichtwellenleiter
- 8: Hauptreflektor
- 9: Durchgang
- 10: Gegenreflektor

## Patentansprüche

1. Bräunungsgerät, das aus einer Liege (2), einem Fluter (3) und ggf. einem Gesichtsbräuner (4) besteht, die einen Bräunungstunnel (5) bilden, wobei aus dem Innenraum der Liege (2), des Fluters (3) und ggf. des Gesichtsbräuners (4) UV-Strahlung in den Bräunungstunnel (5) überführt wird und eine zentrale Strahlenerzeugung (6) vorgesehen ist, die entfernt von den den Bräunungstunnel (5) begrenzenden Strahlenaustrittsflächen (2b, 3b, 4b) angeordnet ist, und wobei die zentrale Strahlenerzeugung (6) mit dem Innenraum (2a, 3a, 4a) der Liege (2), des Fluters (3) und ggf. des Gesichtsbräuners (4) über Lichtwellenleiter (7a, 7b, 7c) verbunden ist, **dadurch gekennzeichnet,** daß die Lichtwellenleiter (7a, 7b, 7c) aus den den Strahlenaustrittsflächen (2b, 3b, 4b) gegenüberliegenden Wandbereichen der Liege (2), des Fluters (3) und ggf. des Gesichtsbräuners (4) vorstehen, wobei jedem Lichtwellenleiterende eine Einrichtung zugeordnet ist, die eine großflächige Ausbreitung des austretenden Strahlenbündels bei gleichmäßiger Verteilung der Strahlung bewirkt.

2. Bräunungsgerät nach Anspruch 1, **dadurch gekennzeichnet,** daß die Einrichtung als Reflektoreinheit ausgebildet ist.

3. Bräunungsgerät nach Anspruch 2, **dadurch gekennzeichnet,** daß die Reflektoreinheit aus einem Hauptreflektor (8) und einem Gegenreflektor (10) besteht.

4. Bräunungsgerät nach Anspruch 3, **dadurch gekennzeichnet,** daß der Hauptreflektor (8) gewölbt und mit seiner konkaven Fläche der Strahlenaustrittsfläche (2b, 3b, 4b) zugewandt ist.

5. Bräunungsgerät nach den Ansprüchen 3 und 4, **dadurch gekennzeichnet,** daß der Durchgang (9) des Lichtwellenleiters (7a, 7b, 7c) durch den Hauptreflektor (8) in dessen optischer Achse angeordnet ist.

6. Bräunungsgerät nach den Ansprüchen 3 - 5, **dadurch gekennzeichnet,** daß der Gegenreflektor (10) in dem vom Hauptreflektor (8) umschlossenen Raum angeordnet, gewölbt und mit seiner konvexen Fläche dem Hauptreflektor (8) zugewandt ist.

7. Bräunungsgerät nach Anspruch 6, **dadurch gekennzeichnet,** daß der Gegenreflektor (10) auf der optischen Achse des Hauptreflektors (8) gegenüber dem Durchgang (9) des Lichtwellenleiters (7a, 7b, 7c) angeordnet ist.

8. Bräunungsgerät nach Anspruch 7, **dadurch gekennzeichnet,** daß der Gegenreflektor (10) in bzw. nahe dem Brennpunkt des Hauptreflektors (8) angeordnet ist.

9. Bräunungsgerät nach Anspruch 3 und mindestens einem der folgenden Ansprüche, **dadurch gekennzeichnet,** daß zwischen Haupt- (8) und Gegenreflektor (10) am Ende des Lichtwellenleiters (7a, 7b, 7c) eine Linse vorgesehen ist.

10. Bräunungsgerät nach Anspruch 1, **dadurch gekennzeichnet,** daß die Einrichtung als Linsensystem ausgebildet ist.

11. Bräunungsgerät nach Anspruch 3 und mindestens einem der folgenden Ansprüche, **dadurch gekennzeichnet,** daß der vom Hauptreflektor (8) und der Strahlenaustrittsfläche (2b) umschlossene Raum der Liege (2) mit einem Gel gefüllt und die Strahlenaustrittsfläche (2b) als transparente formbare Folie ausgebildet ist.

## Claims

1. Tanning apparatus comprising a couch (2), a flooding cover (3) and, optionally, a facial tanner (4), forming a tanning tunnel (5), wherein UV radiation is transmitted from the interior of the couch (2), the flooding cover (3) and, optionally, the facial tanner (4) into the tanning tunnel (5) and a central radiation generator (6) is provided which is arranged at a distance from the radiation emission surfaces (2b, 3b, 4b) delimiting the tanning tunnel (5), and wherein the central radiation generator (6) is connected to the interior (2a, 3a, 4a) of the couch (2), the flooding cover (3) and, optionally, the facial tanner (4) by light wave guides (7a, 7b, 7c), characterised in that the light wave guides (7a, 7b, 7c) project from the wall regions of the couch (2), the flooding cover (3) and, optionally, the facial tanner (4) opposite the radiation emission surfaces (2b, 3b, 4b), the end of each light wave guide being associated with a device which produces large-area propagation of the emergent beam with uniform distribution of the radiation.

2. Tanning apparatus according to claim 1, characterised in that the device is formed as a reflector unit.

3. Tanning apparatus according to claim 2, characterised in that the reflector unit comprises a main reflector (8) and a counter reflector (10).

4. Tanning apparatus according to claim 3, characterised in that the main reflector (8) is curved and its concave surface faces the radiation emission surface (2a, 3a, 4a).

5. Tanning apparatus according to claims 3 and 4, characterised in that the passage point (9) of the light wave guide (7a, 7b, 7c) through the main reflector (8) is arranged on the optical axis thereof.

6. Tanning apparatus according to claims 3 to 5, characterised in that the counter reflector (10) is arranged in the space enclosed by the main reflector (8), is curved and its convex surface faces the main reflector (8).

7. Tanning apparatus according to claim 6, characterised in that the counter reflector (10) is arranged on the optical axis of the main reflector (8) opposite the passage point (9) of the light wave guide (7a, 7b, 7c).

8. Tanning apparatus according to claim 7, characterised in that the counter reflector (10) is arranged in or near the focal point of the main reflector (8).

9. Tanning apparatus according to claim 3 and at least one of the succeeding claims, characterised in that a lens is provided between the main reflector (8) and the counter reflector (10) at the end of the light wave guide (7a, 7b, 7c).

10. Tanning apparatus according to claim 1, characterised in that the device is formed as a lens system.

11. Tanning apparatus according to claim 3 and at least one of the succeeding claims, characterised in that the space in the couch (2) is filled with a gel, the said space being enclosed by the main reflector (8) and the radiation emission surface (2b), and the radiation emission surface (2b) is formed as a transparent, formable film.

## Revendications

1. Appareil de bronzage, constitué d'une couchette (2), d'un canal (3) et éventuellement d'un organe (4) de bronzage du visage, qui forment un tunnel de bronzage (5), dans lequel un rayonnement UV est transmis, depuis l'espace intérieure de la couchette (2), du canal (3) et éventuellement de l'organe (4) du bronzage du visage, dans le tunnel de bronzage (5) et dans lequel est prévu une production centrale (6) de rayons qui est disposée à distance des surfaces (2b, 3b, 4b) de sortie des rayons qui limitent le tunnel de bronzage (5), et dans lequel la production centrale (6) de rayons est reliée à l'espace intérieur (2a, 3a, 4a) de la couchette (2), du canal (3) et éventuellement de l'organe (4) de bronzage du visage par l'intermédiaire de guides d'ondes lumineuses (7a, 7b, 7c), caractérisé par le-fait que les guides d'ondes lumineuses (7a, 7b, 7c) débordent des zones de paroi de la couchette (2), du canal (3) et éventuellement de l'organe (4) de bronzage du visage situées en face des surfaces (2b, 3b, 4b) de sortie des rayons, étant précisé qu'à chaque extrémité de guide d'ondes lumineuses est associé un dispositif qui opère un étalement, sur grande surface, du faisceau de rayons sortants, avec une répartition régulière du rayonnement.

2. Appareil de bronzage selon la revendication 1, caractérisé par le fait que le dispositif est conçu en tant qu'ensemble réflecteur.

3. Appareil de bronzage selon la revendication 2, caractérisé par le fait que l'ensemble réflecteur est constitué d'un réflecteur principal (8) et d'un contre-réflecteur (10).

4. Appareil de bronzage selon la revendication 3, caractérisé par le fait que le réflecteur principal (8) est bombé, avec sa surface concave orientée vers la surface (2b, 3b, 4b) de sortie des rayons.

5. Appareil de bronzage selon les revendications 3 et 4, caractérisé par le fait que le passage (9) du guide d'ondes lumineuses (7a, 7b, 7c) à travers le réflecteur principal (8) est disposé sur l'axe optique de ce dernier.

6. Appareil de bronzage selon les revendications 3-5, caractérisé par le fait que le contre-réflecteur (10) est disposé dans l'espace circonscrit par le réflecteur principal (8), qu'il est bombé, avec sa surface convexe orientée vers le réflecteur principal (8).

7. Appareil de bronzage selon la revendication 6, caractérisé par le fait que le contre-réflecteur (10) est disposée sur l'axe optique du réflecteur principal (8), en face du passage (9) du guide d'ondes lumineuses (7a, 7b, 7c).

8. Appareil de bronzage selon la revendication 7, caractérisé par le fait que le contre-réflecteur (10) est disposé au, ou près du, foyer du réflecteur principal (8).

9. Appareil de bronzage selon la revendication 3 et au moins l'une des revendications suivantes, caractérisé par le fait qu'entre le réflecteur principal (8) et le contre-réflecteur (10) est prévue, a l'extrémité du guide d'ondes lumineuses (7a, 7b, 7c), une lentille.

10. Appareil de bronzage selon la revendication 1, caractérisé par le fait que le dispositif est conçu en tant que système de lentilles.

11. Appareil de bronzage selon la revendication 3 et au moins l'une des revendications suivantes, caractérisé par le fait que l'espace de la couchette (2) circonscrit par le réflecteur principal (8) et par la surface (2b) de sortie des rayons est rempli d'un gel et que la surface (2b) de sortie des rayons est conçue en tant que feuille transparente déformable.
